# EUROPEAN PATENT SPECIFICATION

(11) **EP 3 377 205 B1**
(45) Date of publication and mention of the grant of the patent: **27.04.2022**
(21) Application number: 16825542.0
(22) Date of filing: 18.11.2016
(51) Int. Cl.: B01J 13/14

(54) **A METHOD FOR THE PREPARATION OF PARTICLES WITH CONTROLLED SHAPE AND/OR SIZE**
VERFAHREN ZUR HERSTELLUNG VON PARTIKELN MIT KONTROLLIERTER FORM UND/ODER GRÖSSE
PROCÉDÉ POUR LA PRÉPARATION DE PARTICULES AYANT UNE FORME ET/OU TAILLE RÉGULÉE

(30) Priority: 19.11.2015 BG 11215415
(43) Date of publication of application: 26.09.2018
(73) Proprietor: Sofia University "St. Kliment Ohridski", 1504 Sofia (BG); Smoukov, Stoyan Kostadinov, Cambridge, Cambridgeshire CB4 2QZ (GB)
(72) Inventor: SMOUKOV, Stoyan Kostadinov, Cambridge Cambridgeshire CB4 2QZ (GB); DENKOV, Nikolai Denkov, 1407 Sofia (BG); TCHOLAKOVA, Slavka Stoyanova, 1407 Sofia (BG); LESOV, Ivan Igorov, 1849 Sofia (BG); CHOLAKOVA, Diana Peychova, 1407 Sofia (BG); VALKOVA, Zhulieta Nedyalkova, 6300 Haskovo (BG)
(74) Representative: Radkov, Stoyan Atanassov
(86) International application number: PCT/GB2016/053607
(87) International publication number: WO 2017/085508

(56) References cited:
- WO-A1-2007/060177
- DE-T2- 69 103 807
- US-A1- 2011 275 738
- KUDLA P ET AL: "Phase behavior of liquid-crystalline emulsion systems", ANALYTICAL SCIENCES, THE JAPAN SOCIETY FOR ANALYTICAL CHEMISTRY, US, vol. 349, no. 2, 15 September 2010 (2010-09-15), pages 554-559, XP027169797, ISSN: 0021-9797 [retrieved on 2010-06-01]
- MAKSIMOCHKIN G I ET AL: "Ultrasonic studies of structural transformations and phase transitions in liquid crystal emulsions", ACOUSTICAL PHYSICS, NAUKA/INTERPERIODICA, MO, vol. 57, no. 2, 29 March 2011 (2011-03-29) , pages 264-270, XP019891188, ISSN: 1562-6865, DOI: 10.1134/S1063771011020126
- BONO S ET AL: "Isotropic-to-nematic phase transition of liquid crystals confined in nanoemulsion droplets", EUROPHYSICS LETTERS: A LETTERS JOURNAL EXPLORING THE FRONTIERS OF PHYSICS, INSTITUTE OF PHYSICS PUBLISHING, BRISTOL, FR, vol. 109, no. 2, 29 January 2015 (2015-01-29), page 26004, XP020276200, ISSN: 0295-5075, DOI: 10.1209/0295-5075/109/26004 [retrieved on 2015-01-29]
- FORGIARINI A ET AL: "Formation of Nano-emulsions by Low-Energy Emulsification Methods at Constant Temperature", LANGMUIR, AMERICAN CHEMICAL SOCIETY, US, vol. 17, no. 7, 6 March 2001 (2001-03-06), pages 2076-2083, XP002581274, ISSN: 0743-7463, DOI: 10.1021/LA001362N

## Description

### FIELD OF INVENTION

The present invention relates to a method of preparing particles from emulsions and particularly but not exclusively a method of preparing particles with controlled shape and/or size. The disclosure further relates to particles formed in accordance with the disclosed method.

### PRIOR ART

Currently, the preparation of emulsions with submicron size of the droplets includes two main approaches: high-energy dissipation methods and low-energy dissipation methods. The high-energy dissipation methods require homogenization of oil with water in presence of surfactants. The typical homogenizing equipment includes high-pressure homogenization, rotor-stator, turbulent stirred vessels, ultra-sound, etc. (US 4380503 A; DE3024870A1; DE3024870C2; EP0043091A2; EP0043091A3; EP0043091B1; WO 1995035157 A1; DE69528062D1; DE69528062T2; EP0770422A1; EP0770422A4; EP0770422; US5,843,334; US6,767,637; US2003/0230819). All of these homogenizers are industrially scalable, but they are characterized with very low efficiency, <0.1%. Usually, the >99.9% of the input energy is dissipated as heat. The heat may result in degradation of temperature sensitive components; it can trigger unwanted chemical reactions and wear off the equipment.

Methods, based on low-energy dissipation, could be separated into three sub-groups; solvent exchange methods, phase-inversion method and spontaneous emulsification (US6,599,627; EP1404516A2; US2002/0160109; WO2003/053325A2; WO2003/053325A3; WO2003/053325A8; US5,407,609 A; CA2050911A1; CA2050911C; CN1047223A; DE69024953; DE69024953; DE69024953; EP0471036; EP0471036; EP0471036; EP0471036; WO1990013361; US6,767,637; US2003/0230819; US2013/0011454; CN102821756; WO2011118958; WO2011118958; EP1905505; EP1905505A3; EP1905505; US20080081842; EP1882516; CA2590723A1; CN101121102; DE102006030532; EP1882516; US2008/0004357). The solvent exchange methods are based on the dissolution of the oil phase in an organic solvent. Examples of such solvents are acetone and chloroform. After the dissolution, the oil-solvent solution is mixed with water and the organic solvent is extracted or evaporated. This way, one could prepare nano-particles and nano-emulsions. However, the solvents are usually volatile and/or toxic, which makes them undesirable for pharmaceutical applications and disallows them for food applications.

The spontaneous emulsification leads to the preparation of thermodynamically stable emulsions. Requirements for the preparation of such emulsions is ultra-low interfacial tension (usually in the order 10⁻³ - 10⁻⁶ mN m⁻¹) and vast amount of surfactants, e.g. 10 wt% or more. These emulsions are extremely sensitive to changes in the storage conditions, which limits their usability. In very few cases, one could add oil soluble component (pigments, dyes or drugs) to such system, without causing demulsification.

A pending problem for such spontaneously formed emulsions is the so-called "phase inversion". Often, due to a temperature change or else, the surfactant changes its solubility, which causes the emulsions to change their type: the ones, which are initially water-in-oil become oil-in-water and vice versa. These emulsions are kinetically stable and their average drop size is often smaller than 1 micron. Nevertheless, due to the extreme heating (up to several dozens of degrees Celsius), this method is inappropriate for temperature sensitive components (drugs, proteins, gelatin, etc).

In summary, there is a necessity for the development of a new method for preparation of sub-micron emulsions. The new method should evade the use of high temperature and should be more energy-efficient than the currently existing methods. The new method should be industrially scalable. There are numerous methods, which allow the control over the particle shape (WO2008/031035 A2; US2007/0105972; US8,043,480; WO 2008/100304 A2; US8,420,124; US2007/0054119; WO2008/058297), but these methods allow only ultra-low production volumes. Other methods provide scalability, but are limited in their capabilities for the production of different particle shapes (US4,748,817, EP0266859). Therefore, there is a desire for the development of a new method for preparation of particles with different from spherical shapes.

KUDLA P ET AL: "Phase behavior of liquid-crystalline emulsion systems", ANALYTICAL SCIENCES, THE JAPAN SOCIETY FOR ANALYTICAL CHEMISTRY, US, vol. 349, no. 2, 15 September 2010 (2010-09-15), pages 554-559 discloses a method comprising the steps of preparing an emulsion of molten fatty alcohols in water and cooling said emulsion from 80°C to room temperature whereby the molten fatty alcohols transform into liquid crystals.

### SUMMARY OF INVENTION

The current invention is a method for preparation of particles (e.g. organic and composite particles) with defined shape and/or size. In at least one embodiment, the method allows the control of the shape of the particles (e.g. prepared from organic materials or a combination of organic material(s) and inorganic one(s)). In at least one embodiment, the invention allows the preparation of droplets with submicron size from an emulsion with initial size of the droplets between 5 nm and 1000 microns.

The control over the shape and size of organic particles is important for vast number of applications, including industrial products, such as paints and varnishes, catalyst supports, pharmaceutical drug carriers, as well as the rheological properties and texture of foods and personal care products.

One of the embodiments of the current method is the preparation of particles with pre-defined shape. Their preparation is a two-step process - preparation of an initial emulsion, through mild stirring, membrane emulsification or high-pressure homogenization; and cooling of the emulsion close to the melting point of the drop phase or below it, so the droplets start to deform driven by the formation of, e.g. a plastic crystal phase at the liquid-liquid interface. The plastic phase may be only occur in a subset of the molecules in the droplet (e.g. molecules forming an outer shell of the droplet), and often many of the droplets contain liquid. Such differently shaped droplets could drastically change the rheological properties of the emulsion. Optionally, after transforming into different shapes due to plastic phase transitions, the droplets could be solidified into particles (e.g. by either freezing or by polymerization). The initial emulsion may be prepared in presence of surfactant or a mixture of surfactants, which stabilizes the final emulsion over prolonged periods, such as months and even years.

This method is industrially scalable, and characterizes with low energy consumption compared to high-energy dissipation methods; and may allow much lower concentration of surfactants, compared to low-energy dissipation methods. The method allows applications, where low temperatures or narrow temperature intervals are required. Such are the cases of oil soluble drugs, vitamins, pigments, etc. The method allows shifting the melting point of the particles, via their polymerization or incorporation of functionality of the particles, through dispersion or dissolution of different components in the oil phase. The invention is as disclosed in the appended claims.

### STATEMENTS OF INVENTION

In accordance with a first aspect of the present invention, there is provided a method comprising the steps of:
a. preparing an emulsion of a hydrophobic phase in a hydrophilic phase to form droplets of the hydrophobic phase, where the hydrophobic phase is selected so that during cooling it transforms from a liquid state to a plastic state; and
b. cooling the droplets to a temperature where the hydrophobic phase undergoes a phase transition from a liquid state to a plastic state;
wherein the cooling step b) comprises cooling the droplets at a controlled cooling rate, with a controlled cooling rate between 0.0001 and 5 K per minute.

In one embodiment, the plastic state (e.g. state in which the relative positions of at least a portion of the molecules in the droplets (e.g. portion of molecules forming an outer layer or outer layers of the droplets) start to become fixed but still maintain the ability to change their orientation) is selected from the group of: a rotator phase state (e.g. a plastic crystal state); a polymorphic transition state; and a liquid-crystal state. In the plastic state this portion of molecules (typically a thin outer layer of molecules perhaps in the order of tens to hundreds of molecules in thickness) can counteract the interfacial tension of the emulsion to form energetically favourable states with resulting particle shape change.

Additional steps might be included in the method, such as: a subsequent phase transition from the plastic state to a solid state (e.g. by freezing, polymerization or encapsulation); physical and/or chemical surface modification; functionalization.

In at least one of the embodiments, the emulsion is oil-in-water.

In at least one of the embodiments, the cooling step leads to deformation of the droplets (e.g. into a specific shape (e.g. from a range of obtainable shapes)).

In at least one of the embodiments, the method (e.g. the cooling step b) or a subsequent step (e.g. cooling step and/or a subsequent heating step)) leads to breakage of the droplets into smaller ones than their initial size. In one embodiment after breakage of the droplets the newly formed droplets can continue to transform into different shapes.

In one embodiment the process does not employ a phase inversion step.

In at least one of the embodiments, the cooling could be performed without phase transition of the continuous phase.

In at least one of the embodiments, the initial emulsion could be prepared via microfluidic device.

In at least one of the embodiments, the dispersed or oil phase could form a rotator phase/plastic crystal or liquid crystal.

In at least one of the embodiments, the oil phase is hydrocarbon, e.g. linear alkanes with 10 to 50 carbon atoms in the hydrocarbon chain (C₁₀-C₅₀). Appropriate for room temperature studies are alkanes with 14 to 20 carbon atoms in the hydrocarbon chain.

In at least one of the embodiments, the hydrophobic phase includes one or more of: a linear hydrocarbon, cyclic hydrocarbon, asymmetric alkane, alkene, alkine, alcohol with one or more hydroxyl groups, ester, ether, amine, amide, aldehyde, ketone, fluoro-alkane or a mixture of these compounds.

In at least one of the embodiments, the concentration of the linear hydrocarbon could be between 0.5 and 70 wt% (e.g. between 1 and 70 wt% ) with respect to the emulsion weight and could, for example, be 5, 10, 15, 20, 25, 30, 35, 40, 45, 50, 55, 60, 65, 70 wt.%. Here the term "emulsion" refers to the sum of the masses of the hydrophilic phase (e.g. water), the hydrophobic phase (e.g. oil) and the components dissolved or dispersed in them.

In at least one of the embodiment, the hydrophobic phase comprises a mixture of hydrophobic substances (e.g. a mixture of hydrocarbons).

In at least one of the embodiments, the hydrophobic phase is mixture of two or more alkanes; alkane and oil soluble component(s); or alkane and insoluble component(s), which are dispersed in the oil phase.

In at least one of the embodiments, the hydrophobic phase is mixture of oils, which are not linear alkanes; they might be a combination of linear hydrocarbon, cyclic hydrocarbon, or combining linear and cyclid groups, with alkene, alkine, alcohol with one or more hydroxyl groups, ester, ether, amine, amide, aldehyde, ketone, fluoro-alkane and might contain insoluble components, which are dispersed in the oil phase.

In at least one of the embodiments, the emulsion further comprises at least one oil dispersible component. The at least one oil dispersible component may include surfactants, vitamins, proteins, drugs, drug carriers, solid particles or a mixture of thereof.

In at least one of the embodiments, the at least one oil dispersible component comprises an oil soluble component, e.g. surfactants, molecules forming plastic phases and/or combination thereof.

In at least one of the embodiments, the emulsion contains polymeryzable agent.

In at least one of the embodiments, the emulsion contains nano- or micro-particles. The particles could be inside of the emulsion drops, at their surface or in the water phase.

In at least one of the embodiments, the oil dispersible components (e.g. oil soluble components) could be up to 50 wt% with respect to the mass of the emulsion.

Insoluble components could be up to 50 wt% with respect to the mass of the emulsion.

In at least one of the embodiments, the emulsion contains at least one surfactant.

In at least one of the embodiments, there are one or more surfactants used with hydrophilic-lipophilic balance (HLB) > 14.

In at least one of the embodiments, the substance used for formation of the particles/drops is liquid at room temperature (25°C).

In at least one of the embodiments, the substance used for formation of the particles/drops is solid at room temperature (25°C).

In at least one of the embodiments, the method further comprises a step for melting of the hydrophobic phase before the preparation of the initial emulsion.

In at least one of the embodiments, the method further comprises a step for polymerization of a component (e.g. hydrophobic phase) of the emulsion. In one embodiment polymerization is achieved by the application of UV energy. In another embodiment polymerization is achieved by the application of heat or a reactive substance.

In at least one of the embodiments, the surfactant is water soluble or oil soluble, or there is a combination of water soluble and oil soluble surfactants, e.g. nonionic surfactants, which is soluble in the oil phase and another one, which is soluble in the water phase. The surfactant could be soluble in both phases. Examples of surfactants used in some embodiments are Brij 52, Brij 58, Brij 72, Brij 78, Brij S10, Brij S20, Brij C10, Brij C20, Tween 20, Tween 40, Tween 60, Tween 80, Span 20, Span 40, Span 60, Span 80, Span 85, Lutensol in all of its trade forms, Neodol in all of its trade forms or Enordet in all of its trade forms, as well as other products with the same or similar chemical structure but different trade names.

In at least one of the embodiments, the surfactant is non-ionic. For example, the surfactant may be an ethoxylated surfactant. Examples of surfactants used in some of the embodiments are ethoxylated alcohol, sorbitan ester of their derivatives.

In at least one of the embodiments, the surfactant is ionic. Examples of surfactants used in some of the embodiments are alkyl bromides, alkyl sulfates, alkyl sulfonates, betaine, surfactants with similar functional groups and/or combinations thereof.

In at least one of the embodiments, the surfactant is anionic, e.g. sodium tetradecyl sulfate (C₁₄H₂₉SO₄Na).

In at least one of the embodiments, the surfactant is cationic, e.g. cetyl trimethyl ammonium bromide ((C₁₆H₃₃)N(CH₃)₃Br).

In at least one embodiment, there is ferrofluid, comprising of solid, magnetic nano- and micro particles and oily substance.

In at least one embodiment, there is ceramic material (e.g. in the form of nano- or micro-particles). In one embodiment, the ceramic material is in the form of a shell (e.g. thick shell), which covers the droplets, e.g. core-shell particles with different shapes.

In at least one of the embodiments, a combination of surfactants may be used.

In at least one of the embodiments, the surfactant contains a hydrocarbon chain, which has length close to the one of the used hydrocarbon (oil), whereas it could be shorter than it with up to 4 carbon atoms, equal in length or longer than the one of the component or the mixture of components constrained within the droplets.

In at least one of the embodiments, the surfactant comprises a hydrocarbon chain with a length longer than a hydrocarbon chain of the hydrophobic phase (oil phase).

In at least one of the embodiments, the, the surfactant has a concentration ≤ 5 wt.% with respect to the emulsion, whereas it could be between 0.01 and 5 wt%, e.g. 0.05, 0.5, 1, 1.5, 2, 2.5, 2.75, 3, 3.25, 3.5, 3.75, 4, 4.25, 4.5, 4.75 or 5 wt.%.

In at least one embodiment, the concentration is higher than 5 wt.%, for example higher than 10 wt% and specifically up to 20 wt.%.

In at least one embodiment, there is a chemical reaction undergoing before or during the preparation of the particles.

In at least one of the embodiments, the purity of the surfactant trade product is 95 wt.% or more, e.g. cetyltrimethyl ammonium bromide has purity of at least 99 %.

In at least one of the embodiments, the size of the initial droplets is between 5 nm and 2 mm, e.g. 5 nm and 100 microns, for example between 100 nm and 100 microns (e.g. 50 microns), or for further example between 100 nm and 30 microns.

In at least one of the embodiments, the initial emulsion is mechanically stirred or injected via applied pressure, e.g. via membrane, valve or capillary.

In at least one of the embodiments, the initial emulsion is monodisperse, whereas more than 50 % of the droplets (in number), e.g. 60, 65, 70, 75, 80, 85 or more, are within ±10% of the average size.

In at least one embodiment, the emulsion contains a broad range of sizes, spanning over several characteristic sizes, e.g. between 5 nm and 50 µm, but not limited to this range.

In at least one of the embodiments, the emulsion forming step a) of the method includes preparation of an initial emulsion through any of the following methods: membrane emulsification, homogenization equipment (e.g. high pressure homogenization, rotor-stator homogenization), mechanical stirring (e.g. using stirred vessels, magnetic or non-magnetic stirring devices), mechanical shaking or any combination of these methods.

In at least one of the embodiments, which is not claimed, the final particles are solid or semi-liquid organic particles, prepared via change of the temperature, the latter of which induced change in their shape. The temperature could be set stationary (e.g. with cooling the droplets at a fixed temperature) or changed at a controlled cooling rate, e.g. between 0.0001 and 10 Celsius degrees per minute, including any rate therebetween. Solid organic or composite particles with various shapes could be prepared using this method.

The change in the temperature could be performed at rates different than these rates as well, e.g. with changes up to 30 000 Celsius degrees per second, which occurs at liquid propane jet cooling. The shape of the particles could be changed or frozen in a specific moment.

In one embodiment, the method further comprises a stage of solidifying (e.g. by freezing or polymerization) the droplets (e.g. to form a suspension). The step of solidifying may comprise partially solidifying the droplets by polymerization or phase separation.

In at least one of the embodiments, the method further comprises a stage of freezing the droplets (e.g. after the cooling step) or vitrification of the droplets. In one embodiment, the method comprises a step of freezing or vitrification of the emulsion. Freezing the continuous hydrophilic phase could be performed or not.

In at least one embodiment, the emulsion (e.g. hydrophobic phase) is polymerized (e.g. after the cooling step b)).

In at least one embodiment, the droplets are encapsulated via a (e.g. organic or inorganic) shell.

Encapsulation may take place with or without polymerization. An example of encapsulation of particles without polymerization is the process of polycondensation of tetraethyl orthosilicate (TEOS) on the surface of frozen particles. Different layer-by-layer growth/deposition processes may be possible whilst the particles are frozen/polymerized/encapsulated.

Encapsulation may take place when the droplets are in a non-frozen state or in frozen state (in which case the core may or may not be subsequently melted).

In at least one embodiment, the method comprises functionalization of the droplets. For example, they could include active drug compounds mixed with the oil phase.

Functionalization may take place with or without polymerization. An example of functionalization without polymerization includes surface modification of the particles via inorganic reactions, e.g. silanization of the particles, after their encapsulation with polycondensation of TEOS or similar substances.

The functionalization in liquid state is also possible via addition of oil soluble or oil dispersible components. Such components may be hydrophobic magnetic particles, which remain in the particles (and on their surface) and enable magnetic functionality. Other functionalization is the addition of trimethylolpropane methacrylate in the stearyl methacrylate, which enables (partial) cross-linking of the particles in a pre-defined shape, thus changing the melting point and other properties.

In at least one embodiment, the solid or semi-liquid particles have a smooth surface.

In at least one embodiment, the solid or semi-liquid particles have a rough surface (e.g. in the form of nano- or micro- particles, spikes or polymeric brushes with different length/size and/or density).

In at least one of the embodiments, the emulsion droplets break into smaller ones (e.g. as a result of the cooling step b) and/or of a subsequent step (e.g. cooling step and/or a subsequent heating step)).

The liquid drops could be deformed into different shape, for instance polyhedral, rods with different aspect ratios; prism, with triangular, quadratic or multipolar bases, polygons, etc. The shapes could be continuous or with inscribed holes with geometrical shapes. They might be spherical droplets with fibers coming out of them.

In at least one embodiment of the method, solid particles (e.g. solid organic particles (e.g. with anisotropic shape)) are obtained. The solid particles could be polyhedral, rod-like, fibrilar, prismatic with different geometric bases; they may contain holes with different geometrical shapes and sizes.

In at least one of the embodiments, there is a step of temperature increase above the melting point of the used hydrophobic phase or above the meting point of one of the components in the hydrophobic phase. For example, in one embodiment the temperature of the emulsion in emulsion forming step a) is higher than the melting temperature of the droplets. The latter could be different than the melting point of the bulk hydrophobic material.

In at least one embodiment, the method includes a step of emulsion cooling and/or heating which are performed above the freezing point of the hydrophilic phase.

In at least one of the embodiments, the hydrophilic phase comprises an anti-freezing component (e.g. there is an anti-freezing agent added in the continuous hydrophilic phase), e.g. alcohol, ethylene glycol or glycerol, but not limited to them.

In one embodiment, the concentration of anti-freezing agent is below 30 wt% with respect to the mass of the hydrophilic phase.

In another embodiment, the anti-freezing component is up to 100 vol. % with respect to the volume of the hydrophilic phase.

After the freezing of the (disperse) hydrophobic phase, the temperature may be kept constant or could be increased to melt the particles.

After polymerization of the (disperse) hydrophobic phase, the temperature may be kept constant or could be increased to melt the particles.

The melting temperature of the particles might be different than the melting temperature of the bulk hydrophobic phase, typically lower but in some cases it could be higher as well.

In at least one embodiment, the step of emulsion cooling occurs by achieving (e.g. and maintaining) a temperature below the freezing temperature of the droplets.

In one embodiment, the method further comprises subjecting the emulsion (or suspension formed after solidification of the droplets) to a change (e.g. temperature change) or shock (e.g. mechanical shock) to cause a decrease of droplet size (e.g. by droplet breakup during emulsion cooling and/or by a subsequent increase of the temperature (e.g. whereby complete or partial melting of the droplets is performed).

In at least one embodiment, the method further comprises a (e.g. subsequent) increase of the temperature and complete or partial melting of the droplets/particles is performed. For example, after freezing, the temperature of the emulsion could be increased above the melting point of the droplets and then decreased again. This cycle could be used for the preparation of droplets with submicron size and/or for the preparation of particles with different shapes.

In at least one of the embodiments, the method comprises one or more (e.g. two or more) cycles of cooling and heating of the emulsion (e.g. leading to preparation of smaller particles/droplets and/or evoking further changes in their shape).

In at least one of the embodiments, the method provides emulsions with average diameter of the droplets below one micron.

In at least one of the embodiments, the particles are subsequently isolated from the hydrophilic phase (e.g. and separated into different groups depending upon shape or size). In one embodiment, the particles are separated from the hydrophilic phase via centrifugation, filtration/dialyses or another process of that kind. In another embodiment, the particles are separated using an external field, e.g. gravitational, electric, magnetic.

In at least one of the embodiments, the particles are organic and have anisotropic shape.

In at least one of the embodiments, the method yields submicron droplets or particles (e.g. droplets or particles with a mean diameter of less than 1 micron).

In at least one of the embodiments, the shape of the fluid particles could be controlled via cooling, via using a surfactant and/or via the initial droplet size.

In at least one of the embodiments, the particles are modified by one or more of: polymerization, encapsulations, surface or bulk-modification; and functionalization.

In the case of solid particles formed by the method, modification may take place either before or after solidification.

In at least one embodiment, the particles are modified before or after the cooling step b).

In at least one of the embodiments, the submicron particles could be prepared without using organic solvents or high energy-dissipation method.

The current invention could be used in various applications: pharmacy, foods, cosmetics, electronics, paints and varnishes, catalyst supports, etc.

The current method allows precise control over the shape of the particles, while simultaneously allows high yields.

The current invention is industrially scalable. The method allows the preparation of kilograms and potentially tons of particles over short periods of time, e.g. one day.

In accordance with a second aspect of the present disclosure, there is provided a dispersion of liquid, semi-liquid or solid particles obtained or obtainable from the method of any embodiment of the first aspect of the present invention.

In accordance with a third aspect of the present disclosure, there is provided a solid particle obtained or obtainable from the method of any embodiment of the first aspect of the present invention. Such dispersions and solid particles are disclosed but are not part of the invention.

### DRAWINGS DESCRIPTION

- **Figure 1**: The method allows the preparation of a diverse range of particle shapes: rod-like, with different aspect ratios (1a, 1b); triangular (1c); triangular with inscribed geometrical shapes (d); deformed and/or elongated triangular shapes (e, f); quadrilateral shapes (g, h); quadrilateral shapes with inscribed geometrical shapes (i); hexagonal (j); hexagonal with inscribed geometrical shapes (k, l); and/or polygonal shape (m).
- **Figure 2**: illustrates the experimental set up, used in Example 1. The emulsion [301] is put in a capillary [302]. The capillary is put in a thermostating chamber [303], which is being cooled or heated via circulating liquid [304, 305], while monitored in a microscope [306].
- **Figure 3**: illustrates some of the geometrical shapes of the solid particles, prepared via the current method.
- **Figure 4**: shows the size of the drops in the initial emulsions and after two cycles of freezing and melting of the droplets. Scale, 20 µm, *d₃₂* is the Sauter diameter of the drops.

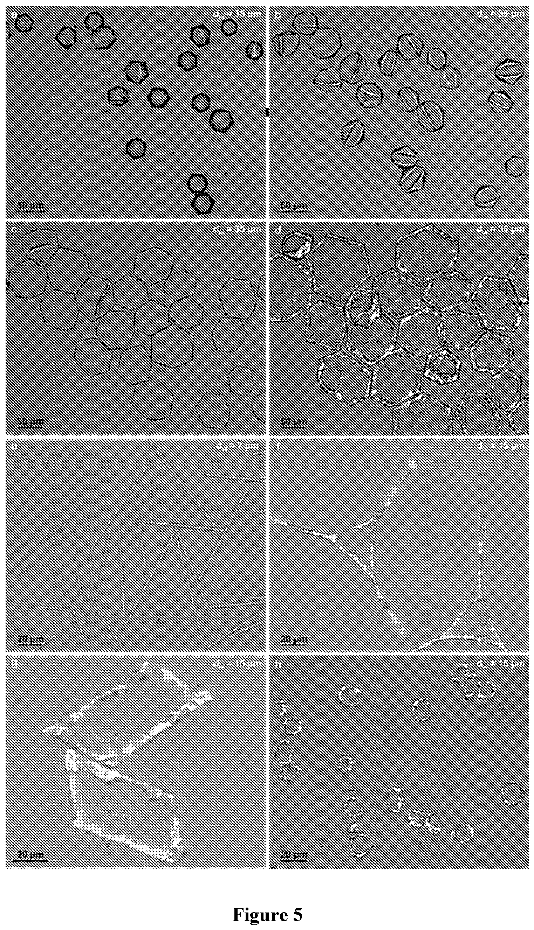

- **Figure 5**: shows pictures of particles, prepared via the current method. The particles are made from hexadecane in the presence of 1.5 wt% surfactant: (a-d) Tween 60, (e) Brij 58 (f-h) Tween 40. (a-d) Consequent phases of deformation of droplets, stabilized with Tween 60. (e) Rod-like particles, after freezing. (f) Frozen triangles with elongated edges. (g) Frozen parallelograms. (g) Toroidal particles. The initial size of the droplets is indicated on the picture and the cooling rates are between 0.5 and 2.0 degrees Celsius.

### TERMINOLOGY

Emulsion is a mixture of two immiscible liquids, whereas one is dispersed in the other in the form of droplets. Generally, the emulsion is made of polar (hydrophilic) phase, e.g. water, and non-polar (hydrophobic) phase, which is called oil. In accordance to the Bancroft rule, when the surfactant, used for the stabilization of the droplets, is more soluble in the water phase, then the expected emulsion type is oil-in-water. Water soluble surfactants have hydrophilic-lipophilic balance (HLB) > 10, for example 30>HLB>14 (e.g. 18>HLB>14).

Immiscible means that after mixing there is more than one component and more than one phase. One of the components could be partially soluble into the other components but at least two separate phases should be present.

The words drop, droplet and particle to be considered interchangeable for the purposes of the current invention.

The word surfactant should be understood as single or multiple surfactants. Surfactants are class of molecules with amphiphilic nature - polar group (head) and non-polar group (tail). The head could be ionic or non-ionic. The tail is usually a hydrocarbon sequence. They could be oil or water soluble. Surfactants with HLB<10 are oil soluble and those with HLB >10 are water soluble.

Initial emulsion is used for the preparation of drops with specific shape and/or breakage into smaller droplets. The initial emulsion consists of oil drops, dispersed in water in presence of surfactant and could be prepared via any other method, including membrane emulsification, high pressure homogenization, rotor-stator homogenization, stirred vessels, magnetic or non-magnetic stirring devices, etc.

Membrane emulsification is a method for injecting one phase into the other by the means of applied pressure (see Examples).

In this invention the expressions rotator phase; plastic crystal; polymorphic transition; and liquid-crystal to be considered synonyms. They are characterized with translational symmetry of the molecules, which however have rotational freedom. (see Sirota, E.B., Herhold, A.B. Transient phase-induced nucleation. Science 283, 529-532 (1999); Ueno, S., Hamada, Y., Sato, K. Controlling Polymorphic Crystallization of n-Alkane Crystals in Emulsion Droplets through Interfacial Heterogeneous Nucleation. Cryst. Growth Des. 3, 935-939 (2003)). Their presence could be detected via X-ray diffraction.

### Examples of surfactant:

**Nonionic surfactants**: Polyoxyethylene glycol alkyl eter: CH₃-(CH₂)₇₋₁₆-(O-C₂H₄)₁₋₂₅-OH, e.g. octa-or penta- ethyleneglycol monodecyl ether; Polyoxypropylene glycol alkyl ethers CH₃-(CH₂)₁₀₋₁₇-(O-C₃H₆)₁₋₂₅-OH; Glycoside alkyl ether CH₃-(CH₂)₁₀₋₁₇-(O-Glucoside)₁₋₃-OH, e.g. decyl- or lauryl-glucoside; Polyoxyethyle glycol octylphenol eters: C₈H₁₇-(C₆H₄)-(O-C₂H₄)₁₋₂₅-OH, e.g. Triton X-100; Polyoxyethylene glycol alkylphenol eters: C₉H₁₉-(C₆H₄)-(O-C₂H₄)₁₋₂₅-OH, for instance Nonoxynol-9; glycerol alkyl esters like glyceryl laurate; Polyoxyethylene glycol sorbitan alkyl esters. Polysorbates; Sorbitan alkyl esters, for example Span; Cocamide DEA, Cocamide MEA, dodecylmethylamine oxide, copolymers of polyoxyethylene glycol and propylene glycol, for instance Poloxamer; and polyoxyethylene amine.

**Cationic surfactants**: Including alkyl trimethyl ammonium salts, e.g. cetyltrimethyl ammonium bromide, cetyltrimethyl ammonium chloride, cetylpyridinum chloride, alkyl dimethyl benzyl ammonium chloride, 5-bromo-5-nitro-1,3-dioxane, dimethyl dioctyl ammonium chloride, cetrimide, dioctyl decyl methyl ammonium bromide, etc.

**Anionic surfactants**: Including ammonium lauryl sulfate, sodium dodecyl sulfate and similar alkyl eter sulfates with different chain length.

**Amphoteric surfactants:** Examples include cocamidopropyl betaine, lauryl betaine, sulfobetaine and their derivatives.

Oil soluble surfactants are some non-ionic surfactants with HLB < 10, e.g. sorbitan esters of fatty acids (polysorbates), such as Span 40, Span 60, Brij 52, etc.

The shape of the particles, prepared through the method presented in this invention, depends on the chemical composition of the dispersed phase (droplets), the initial size of the droplets, the choice of surfactant, the cooling/heating rate or temperature. Additional information is included in the EXAMPLES section.

The controlled rate of cooling/heating is the temperature difference applied by us for a period of time, divided by the time. The rate could be changed, kept constant or could be zero.

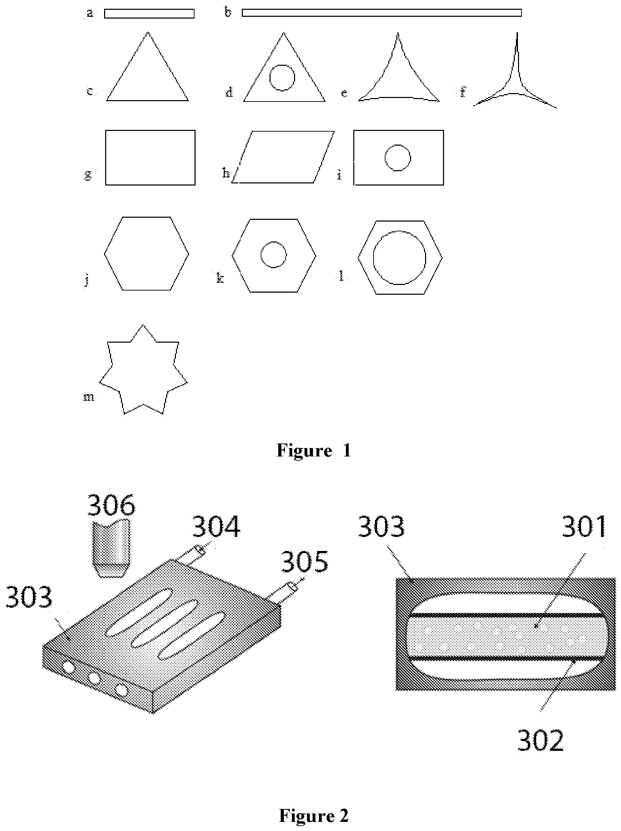

Thermostated vessel - in the current invention thin glass capillaries were used, fitted in a metal plate. There is circulating fluid (Figure 2), which has a temperature, controlled via cryo-thermostate. The method is not limited to capillaries. Vessels could be beakers, cylinders, centrifugal tubes, pipes, etc., as long as their temperature can be changed in a predefined manner.

Rotator phases could be formed from alkanes, alkenes, alkines, alcohols with one or more hydroxyl groups, esters (mono-, di-, tri-, etc.), eters, amides, amines, aldehydes, ketones, nitriles, fluorinated hydrocarbons, mixtures of them (e.g. carboxylic acids, or a mixture of alcohol and aldehyde or ketone), pyrrolidinium salts and derivatives, imidazolium salts and derivatives, etc. The rotator phases must be at least partially insoluble in the hydrophilic phase.

Solid organic particles with anisotropic shape are particles, prepared from any of the aforementioned substances or mixture of substances, which yield shape of the particles different from spherical (which may be the preferred form of small drops in liquids).

Aspect ratio is the relation between the longest projections of the particles, divided by the initial size of the drops, before their deformation. High-aspect ratio is aspect ratio of 5 or more, wherein it could be more than 100.

The current invention uses oil-in-water emulsions for initial emulsions. They are used to produce emulsions with much smaller size of the droplets, e.g. submicron size but not limited to; and/or for control of the particle shape.

The initial emulsion is prepared via any other method. The emulsion contains oil droplets, dispersed in water or in water-containing solution or in mixture of hydrophilic phases. The deformation of the droplets depends on the applied temperature, the oil chosen, the drop size, waiting time, etc.

The choices of surfactant and oil define if the drops are going to break into smaller ones, but are not the only limiting factors. The drop breakage could occur during the cooling or during the melting of already frozen or deformed particles. The temperature of breakage is system specific and it could be higher or lower than the melting/freezing temperature of the bulk phase.

The method requires different temperatures and temperature intervals, depending on the oil, surfactant, drops size, etc. For instance, the preparation of tetradecane droplets with different shapes requires working between 273 and 280 K, while for hexadecane it is necessary to work in between 282 and 291 K and for eicosane-between 303 and 308 K for droplets with the same size and surfactant.

One of the potential applications is the enhanced control over rheological properties of emulsions and suspensions. Using the method described here allowed preparation of particles with high aspect ratios, which could increase the viscoelastic response several orders of magnitude even at low concentrations of the dispersed phase.

Other applications include pharmacy and food sectors. Both sectors often use temperature-sensitive components, such as vitamins, which should not be heated. This method allows working at ambient or lower temperatures and narrow temperature intervals.

The method does not require the use of volatile solvents; it has a high yield and requires low energy consumption compared to conventional shear methods.

### EXAMPLES

Alkanes, used in the current invention are purchased from Sigma-Aldrich and have analytical purity, ≥ 99%. Additional purification of alkanes was performed by the means of silicagel column (Florisil). The interfacial tension of the alkanes used in the current study was ≥ 50 mN/m, depending on the specific hydrocarbon used. In presence of surfactants the interfacial tension was between 2 and 10 mN/m at temperatures close to the freezing temperature of the drops.

Emulsions were prepared with membrane emulsification in presence of 1.5 wt% water soluble surfactant. The amount of surfactant was calculated with respect to the water phase. The oil droplets were generated by the means of glass membranes (Shiratzu porous glass). Membranes had different size of monodisperse pores - generally: 1, 2, 3, 5 or 10 µm. In the membrane there was oil phase - upon applying pressure, the oil started moving through the membrane in the water phase, thus forming monodisperse droplets of oil-in-water. The surfactants dissolved in the water phase were selected to have HLB > 14, e.g. Brij 58 has HLB of 15.7; Brij 78 - HLB = 15.3; Tween 40 has HLB = 15.5; and Tween 60 has HLB 14.9.

Emulsions were put in capillaries - 50 mm long, 1 mm wide and 0.10 mm high. The capillaries were put in a thermostated vessel, consisting of a metal plate with water circulating through it. The vessel is connected to a cryo-thermostate (Julabo CF30), allowing high precision temperature control (accuracy ± 0.2 °C).

During the cooling/heating of the emulsions a microscope Axioplan or AxioImager.M2m (Zeiss, Germany) was used in transmitted white, polarized light. The microscopes were equipped with λ plate, set at 45° in between the analyzer and the polarizer. The observations were held by the means of long-distance objective with 20, 50 or 100 times magnification. The size of the drops and particles was determined from the microscopic images.

The surfactants are a class of substances, consisting of a polar part (head) and non-polar part (tail). The tail usually consists of a hydrocarbon segment, while the head consist of a functional group, which could be either ionic or non-ionic. As a result of its structure, the surfactant has amphiphilic nature - hydrophobic tail and hydrophilic head. As a rule, surfactants with tails similar or longer than the used hydrocarbon (in the case of alkanes) have a higher freezing temperature than the alkane itself. As a result, during the cooling of the emulsions the surface "hardens" and changes the shape of the droplets.

The cooling rate affects the observed phenomenon significantly. At cooling rates lower than 5 degrees Celsius, the emulsion droplets change their shape significantly. For example, the emulsions prepared in the presence of 1.5 wt.% Brij 58 and hexadecane form polyhedra initially. The polyhedra gradually evolve in series of different shapes: hexagonal prisms, then quadrupolar prisms, elongated quadrupolar prisms with high aspect ratio and in the end they become fibers. Each of the stages of the drop shape evolution could be used for preparation of particles, either by freezing or via vitrification. The yield of the different shapes, however, differs: Brij 58 enables yields as high as 75 ± 5 % for quadrupolar prisms and 25 ± 5 % for triangular ones; or 90± 5 % for high-aspect ratio quadrupolar prisms; or 90 ± 5 % for fibrilar structures, depending on the different ways of preparation. Tween 60 allows preparation of more than 90 % rod-like particles.

The shape of the particles depends on the size of the droplets in the emulsions. At higher rates of cooling, e.g. 5 K/min, depending on the surfactant used, the largest drops often freeze without shape transformations.

### Example 1 - Preparation of particles with different aspect ratios.

The current example demonstrates the preparation of solid particles with different aspect rations, as illustrated in Figure 1. The nonionic surfactant, Tween 40, is dissolved into water. Its concentration is 1.5 wt. % with respect to the mass of water. Then hexadecane droplets with diameter 15 µm are injected into the water phase. The concentration of the droplets is 1 vol. % with respect to the whole amount of emulsion. The emulsion [301] is put in a capillary [302] and put in thermostated chamber [303]. There is cooling liquid which circulates throughout the vessel [304, 305].

The initial temperature is 298 K and the cooling rate is 1.4 K/min. As a result the drops deform to hexagonal prisms and then freeze. Their aspect ratio (final-to-initial length ratio) is 4. The initial temperature is 298 K and the cooling rate is 0.16 K/min. As a result the drops deform to rod-like or fibrilar particles. Their aspect ratio (final-to-initial length ratio) is higher than 50. The yield is around 90% in number of particles for both cooling rates.

### Example 2 - Preparation of submicron drops and/or particles (not according to the invention)

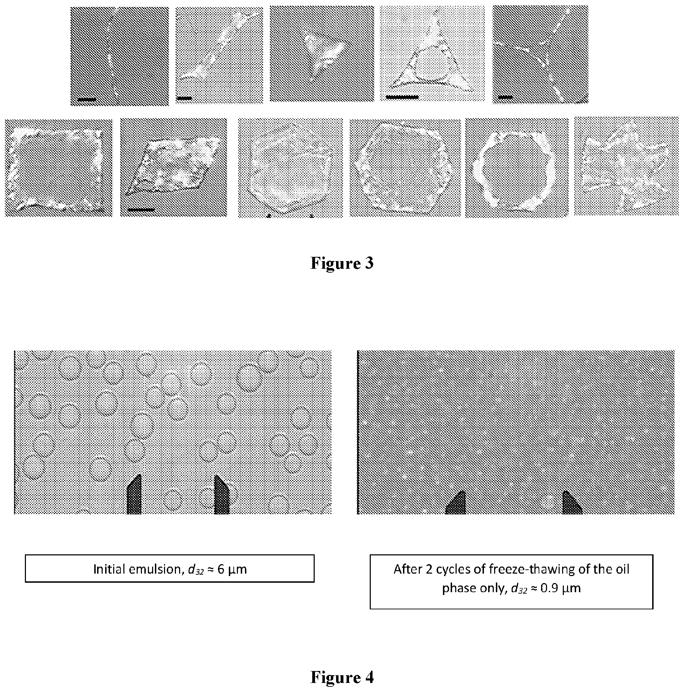

This example demonstrates the drop-size reduction, which is illustrated in Figure 4. 0.6 wt.% Brij 58 is dissolved in water and 0.4 wt.% Brij 52 is dissolved in hexadecane. The hexadecane is dispersed in water in volume ratio 1:3, through membrane emulsification. The emulsions are cooled down from 298 to 278 K in a fridge and then heated back up to 298 K. After two cycles the final drop size 0.9 µm in diameter. Depending on the final temperature, the droplets could be liquid or solid.

### Example 3 - Polymerization (not according to the invention)

The current example demonstrates the preparation of polymerized particles with different geometrical shapes, as demonstrated in Figure 1. The nonionic surfactant, Tween 40, is dissolved into water. Its concentration is 0.15 wt. % with respect to the mass of water. Then stearyl methacrylate droplets with diameter 10 µm are injected into the water phase. The concentration of the droplets is 1 vol. % with respect to the whole amount of emulsion. The emulsion [301] is mixed with water soluble component - α-ketoglutaric acid, e.g. 1.75 wt.% with respect to the water phase; then put in a capillary, and finally - put in thermostated chamber.

The initial temperature of the emulsion is 298 K and the temperature in the cooling chamber is 292±3 K. As a result from the initial spherical drops undergo a transition into hexagonal prisms within 0 to 15 minutes or triangular prisms, when t > 10min. The liquid prisms could be polymerized via irradiation with UV light at 365 nm, or left to change shape and then polymerized. Yield was more than 80 % hexagonal prisms (by number of drops converted in prisms) or more than 50% triangular prisms.

### Example 4 - Composite formation (not according to the invention)

Ferrofluid or hydrophobic ceramic nano-particles with concentration 2 wt% are dispersed in stearyl methacrylate. Then procedure in example 3 is followed.

### Example 5 - Tuning surface chemistry and morphology (growing spikes or brushes) (not according to the invention)

The nonionic surfactant, Tween 40, is dissolved into water. Its concentration is 10-16 wt. % with respect to the mass of water. Then stearyl methacrylate droplets with diameter 35 µm are injected into the water phase. The concentration of the droplets is 1 vol. % with respect to the whole amount of emulsion. The emulsion [301] is mixed with water soluble component - α-ketoglutaric acid, e.g. 0.5 wt.% with respect to the water phase; then put in a capillary [302], and finally - put in thermostated chamber [303].

The initial temperature of the emulsion is 298 K and the temperature in the cooling chamber is 308±3 K. The liquid drops are polymerized via irradiation with UV light at 365 nm for 30-60 min with UV LED. Then, emulsion/suspension is cooled down to 292±3 K and left for few hours. Spike with different size and density are grown based on time for cooling. For example, 10 wt% Tween 40 gives at least 5 µm spikes (brushes) within 5 hours of waiting.

### Example 6 - Organic encapsulation of oil-soluble components (not according to the invention)

The nonionic surfactant, Tween 40, is dissolved into water. Its concentration is 0.3 wt. % with respect to the mass of water. Then stearyl methacrylate droplets with diameter 10 µm are injected into the water phase. The methacrylate droplets might contain any of the listed oil soluble components, but not limited to them:
Trimethylolpropane methacrylate up to 10 wt%, for example 5 wt%
Methacrylic acid up to 25 wt%, and for example 5 wt%
Acrylic acid up to 25 wt%, and for example 5 wt%

The emulsion is mixed with water soluble component - α-ketoglutaric acid, e.g. 1.75 wt.% with respect to the water phase; then put in a capillary, and finally - put in thermostated chamber.

The initial temperature of the emulsion is 298 K and the final temperature depends on the amount of added components and the component type: typically between 273 and 353 K. The liquid drops are polymerized via irradiation with UV light at 365 nm for 15-30 min with UV.

## Claims

1. A method comprising the steps of:
a. preparing an emulsion of a hydrophobic phase in a hydrophilic phase to form droplets of the hydrophobic phase, where the hydrophobic phase is selected so that during cooling it transforms from a liquid state to a plastic state; and
b. cooling the droplets to a temperature where the hydrophobic phase undergoes a phase transition from a liquid state to a plastic state;
wherein the cooling step b) comprises cooling the droplets at a controlled cooling rate, with a controlled cooling rate between 0.0001 and 5 K per minute.

2. The method according to claim 1, wherein the hydrophobic phase contains one or more of the following substances or classes of substances: linear hydrocarbon, cyclic hydrocarbon, asymmetric alkane, alkene, alkyne, alcohol with one or more hydroxyl groups, ester, ether, amine, amide, aldehyde, ketone, fluoro-alkane.

3. The method according to claim 2, wherein the linear hydrocarbon molecules contain between 10 and 50 carbon atoms.

4. The method according to claim 3, wherein the hydrophobic phase is between 0.1 and 70 wt %, with respect to the emulsion, and wherein the hydrophobic phase comprises a mixture of hydrophobic substances.

5. The method according to claim 4, wherein the emulsion further comprises at least one oil dispersible component, wherein the concentration of oil dispersible component is up to 50 wt % with respect to the weight of the emulsion.

6. The method according to any one of claims 1 to 5, wherein the emulsion contains at least one surfactant, including surfactants from the following classes, non-ionic surfactant, such as non-ionic ethoxylated surfactant or ionic surfactant.

7. The method according to claim 6, wherein the ionic surfactant comprises one or a combination of alkyl trimethyl ammonium salts, alkyl bromide, alkyl sulfate, alkyl sulfonate, or betaine.

8. The method according to claim 6 or 7, wherein the concentration of the one or more surfactants is lower than 5 wt. % with respect to the weight of the emulsion.

9. The method according to claim 6 or 7, wherein the one or more surfactants comprise a hydrocarbon chain with a length up to 4 carbon atoms shorter than a hydrocarbon chain of the hydrophobic phase.

10. The method according to anyone of claims 1 to 9, wherein the method includes preparation of an initial emulsion with an initial size of the droplets between 5 nm and 2 mm through membrane emulsification, mechanical stirring, mechanical shaking or using homogenization equipment.

11. The method according to any one of claims 1 to 10, further comprising a stage of solidifying the droplets, wherein the droplets are solidified by freezing or polymerization and/or further comprising a step of cooling and/or heating performed above the freezing point of the hydrophilic phase, wherein the hydrophilic phase comprises an anti-freezing component, and wherein the anti-freezing component is up to 95 vol. % with respect to the emulsion volume.

12. The method according to any one of claims 1 to 11, wherein the method further comprises one or more of functionalization of the droplets or encapsulation of the droplets.

13. The method according to claim 12, wherein the droplets are isolated from the hydrophilic phase.

## Patentansprüche

1. Verfahren, umfassend die Schritte:
a. Herstellen einer Emulsion einer hydrophoben Phase in einer hydrophilen Phase, um Tröpfchen der hydrophoben Phase zu bilden, wobei die hydrophobe Phase so ausgewählt wird, dass sie sich bei Abkühlen von einem flüssigen Zustand in einen plastischen Zustand umwandelt; und
b. Abkühlen der Tröpfchen auf eine Temperatur, bei der die hydrophobe Phase einen Phasenübergang von einem flüssigen Zustand zu einem plastischen Zustand durchläuft;
wobei der Kühlschritt b) Kühlen der Tröpfchen mit einer gesteuerten Kühlrate mit einer gesteuerten Kühlrate von zwischen 0,0001 und 5 K pro Minute umfasst.

2. Verfahren gemäß Anspruch 1, wobei die hydrophobe Phase einen oder mehrere der folgenden Stoffe oder Stoffklassen enthält: linearen Kohlenwasserstoff, cyclischen Kohlenwasserstoff, asymmetrisches Alkan, Alken, Alkin, Alkohol mit einer oder mehreren Hydroxygruppen, Ester, Ether, Amin, Amid, Aldehyd, Keton, Fluoralkan.

3. Verfahren gemäß Anspruch 2, wobei die linearen Kohlenwasserstoffmoleküle zwischen 10 und 50 Kohlenstoffatome enthalten.

4. Verfahren gemäß Anspruch 3, wobei die hydrophobe Phase zwischen 0,1 und 70 Gew.-%, bezogen auf die Emulsion, darstellt und wobei die hydrophobe Phase ein Gemisch von hydrophoben Stoffen umfasst.

5. Verfahren gemäß Anspruch 4, wobei die Emulsion ferner wenigstens eine öldispergierbare Komponente umfasst, wobei die Konzentration an öldispergierbarer Komponente bis zu 50 Gew.-%, bezogen auf das Gewicht der Emulsion, beträgt.

6. Verfahren gemäß einem der Ansprüche 1 bis 5, wobei die Emulsion wenigstens ein Tensid enthält, einschließlich Tenside aus denn folgenden Klassen, nichtionisches Tensid, wie z. B. nichtionisches ethoxyliertes Tensid, oder ionisches Tensid.

7. Verfahren gemäß Anspruch 6, wobei das ionische Tensid eines oder eine Kombination von Alkyltrimethylammoniumsalzen, Alkylbromid, Alkylsulfat, Alkylsulfonat oder Betain umfasst.

8. Verfahren gemäß Anspruch 6 oder 7, wobei die Konzentration des einen oder der mehreren Tenside niedriger als 5 Gew.-%, bezogen auf das Gewicht der Emulsion, ist.

9. Verfahren gemäß Anspruch 6 oder 7, wobei das eine oder die mehreren Tenside eine Kohlenwasserstoffkette mit einer Länge von bis zu 4 Kohlenstoffatome kürzer als eine Kohlenwasserstoffkette der hydrophoben Phase umfassen.

10. Verfahren gemäß einem der Ansprüche 1 bis 9, wobei das Verfahren Herstellen einer Ausgangsemulsion mit einer Ausgangsgröße der Tröpfchen von zwischen 5 nm und 2 mm durch Membranemulgierung, mechanisches Rühren, mechanisches Schütteln oder Verwenden einer Homogenisierungsvorrichtung umfasst.

11. Verfahren gemäß einem der Ansprüche 1 bis 10, ferner umfassend eine Stufe des Verfestigens der Tröpfchen, wobei die Tröpfchen durch Einfrieren oder Polymerisation verfestigt werden, und/oder ferner umfassend einen Schritt des Kühlens und/oder Erhitzens, der über dem Gefrierpunkt der hydrophilen Phase durchgeführt wird, wobei die hydrophile Phase eine Gefrierschutzkomponente umfasst und wobei die Gefrierschutzkomponente bis zu 95 Vol.-%, bezogen auf das Emulsionsvolumen, bildet.

12. Verfahren gemäß einem der Ansprüche 1 bis 11, wobei das Verfahren ferner eines oder mehrere von Funktionalisierung der Tröpfchen und Verkapselung der Tröpfchen umfasst.

13. Verfahren gemäß Anspruch 12, wobei die Tröpfchen von der hydrophilen Phase isoliert werden.

## Revendications

1. Procédé comprenant les étapes de :
a. préparation d'une émulsion d'une phase hydrophobe dans une phase hydrophile pour former des gouttelettes de la phase hydrophobe, où la phase hydrophobe est choisie de sorte que pendant un refroidissement elle se transforme d'un état liquide à un état plastique ; et
b. refroidissement des gouttelettes à une température où la phase hydrophobe subit une transition de phase d'un état liquide à un état plastique ;
l'étape de refroidissement b) comprenant le refroidissement des gouttelettes à une vitesse de refroidissement régulée, avec une vitesse de refroidissement régulée comprise entre 0,0001 et 5 K par minute.

2. Procédé selon la revendication 1, la phase hydrophobe contenant l'une ou plusieurs parmi les substances suivantes ou classes de substances :
hydrocarbure linéaire, hydrocarbure cyclique, alcane asymétrique, alcène, alcyne, alcool comportant un ou plusieurs groupes hydroxyle, ester, éther, amine, amide, aldéhyde, cétone, fluoro-alcane.

3. Procédé selon la revendication 2, les molécules d'hydrocarbure linéaire contenant entre 10 et 50 atomes de carbone.

4. Procédé selon la revendication 3, la phase hydrophobe étant présente en une quantité comprise entre 0,1 et 70 % en poids, par rapport à l'émulsion, et la phase hydrophobe comprenant un mélange de substances hydrophobes.

5. Procédé selon la revendication 4, l'émulsion comprenant en outre au moins un composant dispersible dans l'huile, la concentration de composant dispersible dans l'huile allant jusqu'à 50 % en poids par rapport au poids de l'émulsion.

6. Procédé selon l'une quelconque des revendications 1 à 5, l'émulsion contenant au moins un tensioactif, y compris des tensioactifs parmi les classes suivantes, un tensioactif non ionique, tel qu'un tensioactif éthoxylé non ionique ou un tensioactif ionique.

7. Procédé selon la revendication 6, le tensioactif ionique comprenant l'un ou une combinaison de sels d'alkyltriméthylammonium, bromure d'alkyle, sulfate d'alkyle, sulfonate d'alkyle, ou bétaïne.

8. Procédé selon la revendication 6 ou 7, la concentration du ou des tensioactifs étant inférieure à 5 % en poids par rapport au poids de l'émulsion.

9. Procédé selon la revendication 6 ou 7, le ou les tensioactifs comprenant une chaîne hydrocarbonée dotée d'une longueur jusqu'à 4 atomes de carbone plus courte qu'une chaîne hydrocarbonée de la phase hydrophobe.

10. Procédé selon l'une quelconque des revendications 1 à 9, le procédé comprenant la préparation d'une émulsion initiale dotée d'une taille initiale des gouttelettes comprise entre 5 nm et 2 mm par le biais d'une émulsification par membrane, d'un brassage mécanique, d'une agitation mécanique ou l'utilisation d'un appareil d'homogénéisation.

11. Procédé selon l'une quelconque des revendications 1 à 10, comprenant en outre un stade de solidification des gouttelettes, les gouttelettes étant solidifiées par congélation ou polymérisation et/ou comprenant en outre une étape de refroidissement et/ou de chauffage réalisée au-dessus du point de congélation de la phase hydrophile, la phase hydrophile comprenant un composant anti-congélation, et le composant anti-congélation étant présent jusqu'à 95 % en volume par rapport au volume d'émulsion.

12. Procédé selon l'une quelconque des revendications 1 à 11, le procédé comprenant en outre l'une ou plusieurs parmi une fonctionnalisation des gouttelettes et une encapsulation des gouttelettes.

13. Procédé selon la revendication 12, les gouttelettes étant isolées de la phase hydrophile.
